# EUROPEAN PATENT APPLICATION

(11) **EP 2 545 858 A1**
(43) Date of publication of application: **16.01.2013**
(21) Application number: 11753361.2
(22) Date of filing: 08.03.2011
(51) Int. Cl.: A61B 17/02, A61B 17/00

(54) **SURGICAL RETRACTOR**

(30) Priority: 08.03.2010 JP 2010051077
(71) Applicant: Kawamoto Corporation, Osaka 540-0022 (JP)
(72) Inventor: HAMAGUCHI, Takeyuki, Osaka-shi Osaka 540-0022 (JP)
(74) Representative: Müller-Boré & Partner Patentanwälte
(86) International application number: PCT/JP2011/055376
(87) International publication number: WO 2011/111703

(57) **Abstract**

An excluder (90) inserted into a human body for surgery. The excluder body (91) of the excluder (90) is formed by compressing and shaping a water-absorbing dilatant material so that expansion stress in the direction opposite the direction of the compression remains therein and so that the value of the residual expansion stress varies from portion to portion. Weld sections (92) consisting of a thermoplastic resin are provided to the surfaces (side surfaces (91b, 91d)) facing the direction in which the residual expansion stress is acting. When supplied with water, the excluder body (91) swells large at the center portion (C) thereof at which the expansion stress is high and the excluder body (91) swells small at both the end portions (E) thereof at which the expansion stress is low. The weld sections (92) do not extend, and this controls the shape of the excluder (90) so that, after swelling, the excluder has a curved shape.

## Description

### TECHNICAL FIELD

The present invention relates to a surgical excluder that is used for excluding an organ other than a target organ in a surgery.

### BACKGROUND ART

Surgical operations are categorized broadly into surgeries involving wide incision of an abdominal region, a chest region, or the like (hereinafter, may be referred to as laparotomy) and endoscopic surgeries performed through a small hole opened in an abdominal region or the like.

Such a surgery involves excision, extirpation, or the like of an organ, and may be possibly interrupted by an organ other than a target organ (an untargeted organ) and is thus difficult to be performed. Therefore desired is that such an untargeted organ interrupting a view is excluded to secure a visual field for the target organ.

In order to exclude an untargeted organ, there have been a method of inclining the posture of a patient to exclude the organ due to the gravity, as well as a method of pushing away the untargeted organ with use of an excluder made of sponge or the like.

As such a sponge excluder for laparotomy, there is known an excluder that is made of cellulose sponge and has a thick flat plate shape. Because the cellulose sponge excluder is softened by absorbing fluid, the untargeted organ can be excluded gently. In addition, the cellulose sponge excluder, which is swollen by absorbing fluid, exerts appropriate repulsive force and has a weight of a certain degree. Also with use of its rough surface, such a swollen cellulose sponge excluder is capable of excluding the untargeted organ only by the provision thereof, with no need for an operator or an assistant to press the excluder.

There has been proposed an endoscopic excluder for use in an endoscopic surgery, including a rubber sheet, and a ring frame made of a super-elastic alloy and attached to surround the rubber sheet (refer to Patent Document 1, for example). In use thereof, this endoscopic excluder is flattened out into a thin and straight shape, is inserted through a trocar, and is then expanded in width in a body cavity by restoring force of the ring frame to exclude an organ.

There is also proposed an endoscopic excluder (a support forceps) that is provided with a balloon at a tip end thereof (refer to Patent Document 2, for example). This excluder is inserted into a body cavity through a trocar with the balloon being folded and accommodated in a cylindrical haft. The haft is then slid to expose the balloon, into which gas is injected, so that the expanded balloon excludes or supports an organ. After the use thereof, the balloon is degassed and folded so as to be accommodated in the cylindrical haft, and the excluder is removed out of the trocar.

Although each of the excluders described in Patent Documents 1 and 2 is configured to exclude an organ by means of the rubber sheet or the balloon, which are relatively soft, the frame or the haft to support the rubber sheet or the balloon is solid. Accordingly, the cellulose sponge excluder is superior because such an excluder gently excludes an organ in a body cavity.

Moreover, the endoscopic excluder according to Patent Documents 1 and 2 need manpower for supporting the excluder to keep the excluding state. To the contrary, the cellulose sponge excluder needs no manpower for keeping the excluding state, which is another superior feature.

### PRIOR ART DOCUMENTS

### PATENT DOCUMENTS

Patent Document 1: JP-A-2003-164459
Patent Document 2: JP-A-2005-253916

### SUMMARY OF THE INVENTION

### PROBLEM TO BE SOLVED BY THE INVENTION

As described above, the cellulose sponge excluder has various advantageous features, while there is only known the rectangular flat plate shape as a mode thereof.

The applicant of the present invention has newly devised and already filed an endoscopic excluder for use in an endoscopic surgery (International Application No. PCT/JP2009/064777). The endoscopic excluder according to this application already filed includes an excluder body that is made of a water-absorbing dilatant material (such as cellulose sponge), has a bar shape, and is compressed and shaped so as to have a transverse section (a section perpendicular to the longitudinal direction) smaller than the section of the cylindrical inner cavity of a trocar. This endoscopic excluder can be inserted into a body cavity through the trocar, and upon supply of water, can absorb water to be swollen and increased in size in the body cavity, thereby being capable of satisfactory excluding an organ.

As described above, the invention of the above application already filed can exert the excellent effects, while only a rectangular flat plate shape is exemplified as a shape after being swollen.

In consideration of further usability in surgeries, it is an object of the present invention to provide a surgical excluder that can be handled more easily.

### SOLUTIONS TO THE PROBLEM

In accordance with an aspect of the present invention, a surgical excluder to be inserted into a body during a surgery comprises an excluder body configured by compressing and shaping a water-absorbing dilatant material so that expansion stress in a direction opposite to a direction of compression remains, the residual expansion stress having values varied from portion to portion. The surgical excluder according to the present invention is applicable in any of endoscopic surgery and laparotomy (inclusive of thoracotomy). The "water-absorbing dilatant material" has a property of expanding by absorption of water, and examples thereof include cellulose sponge. The "expansion stress" indicates restoring force of swelling in the direction opposite to the direction of compression.

As described above, the excluder body is configured by compressing and shaping a water-absorbing dilatant material. Accordingly, when water is supplied thereto, the excluder body is swollen and increased in size. The expansion stress is varied from portion to portion. There are thus portions swollen remarkably and portions swollen slightly depending on the differences of the values (high or low) of the expansion stress. The swollen excluder body has a shape different from the shape before being swollen (in the compressed and shaped state) due to the difference. In other words, the excluder body is not swollen into a similar shape but into a different shape.

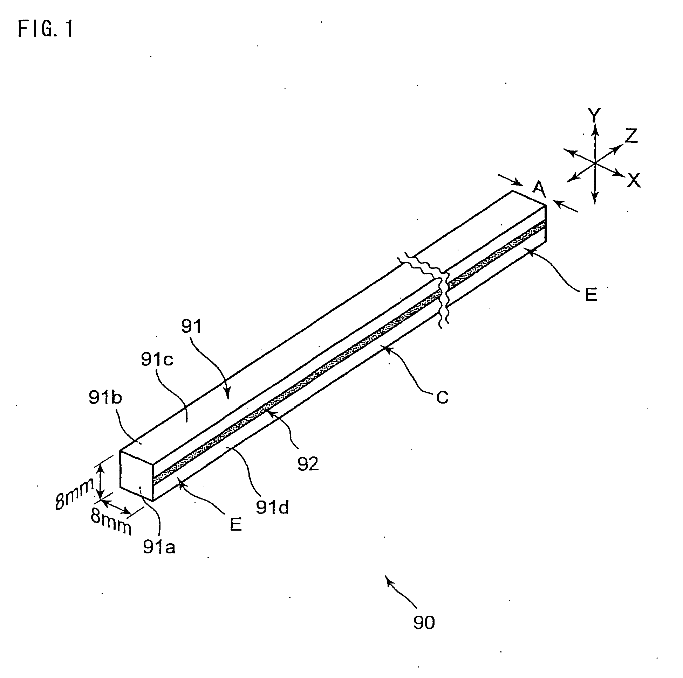

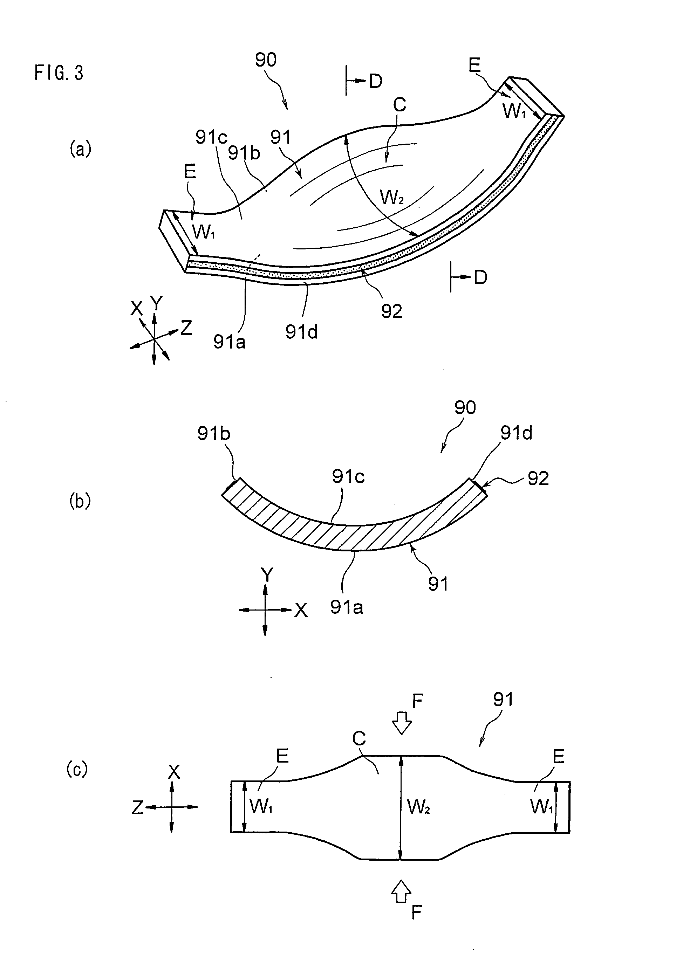

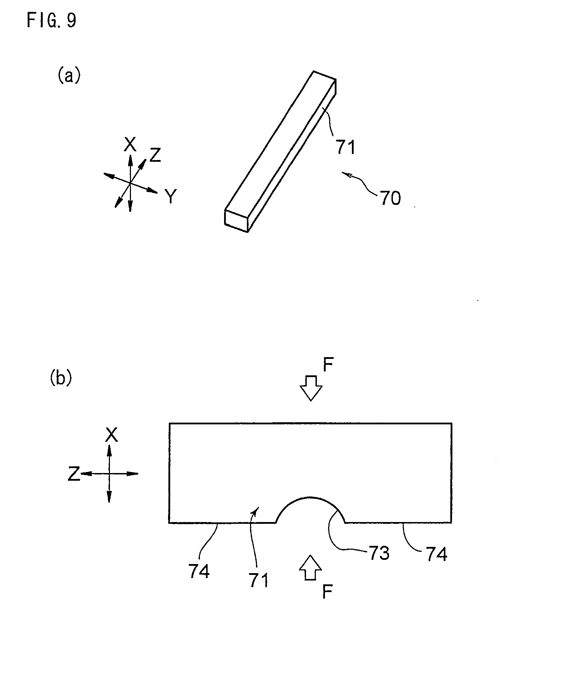

Typical examples of the shape after being swollen include a flat plate shape with a widened center portion (see Fig. 3(c) to be referred to later), and a rectangular flat plate shape having a partial cutout (see Fig. 9(b) to be referred to later). However, the swollen excluder is not limited to these exemplary shapes, but may have various shapes. Examples of the shape before being swollen (in the compressed and shaped state) include a bar shape (see Fig. 1 to be referred to later), a cubic shape, and a rectangular parallelepiped shape.

In a case of being used as an endoscopic excluder, the excluder needs to be inserted through a trocar and is thus limited by the shape of a section of a cylindrical inner cavity of the trocar. As to this limitation, even when the excluder body is formed by compressing and shaping so as to be inserted through the trocar, such an excluder can be designed to have a desired shape after being swollen by varying the expansion stress from portion to portion as described earlier.

When the shape before being swollen (in the compressed and shaped state) is formed into the bar shape, the rectangular parallelepiped shape, or the like regardless of the shape after being swollen, the excluder can be shaped so as to be inserted through the trocar as an endoscopic excluder, or can be accommodated in a small space for storage as an excluder for laparotomy. The excluder is deformed into a different shape as desired after being swollen.

The surgical excluder preferably comprises a weld section made of a thermoplastic resin on at least one of pressure applied surfaces to which pressure is applied upon compressing and shaping the excluder body, the weld section restraining the excluder body. In other words, the weld section made of a thermoplastic resin is preferably provided across the portions of the excluder body to each of which the expansion stress of a different value is applied.

The excluder body thus compressed and shaped is swollen and increased in size when water is supplied thereto, while the weld section is not extended (not swollen) even if water is supplied thereto. Accordingly, the weld section serves to pull the swollen portion (suppress swelling). As a result, the excluder after being swollen can be provided with a curved portion.

In the excluder body comprising the weld section, the pressure applied surfaces of the excluder body each have a width preferably not less than 5 mm. More specifically, when the excluder having been compressed and shaped has a hexahedral shape, and the direction of compression and the direction opposite to the direction of compression are each referred to as an X direction, one of directions perpendicular to the X direction is referred to as a Y direction, and a direction perpendicular to the X direction and the Y direction is referred to as a Z direction, the surfaces along the YZ plane (the pressure applied surfaces) of the excluder body each have a width preferably not less than 5 mm.

Upon supplying water to the excluder, if two surfaces (hereinafter, may be referred to as a front surface and a rear surface) along the XZ plane of the excluder body are supplied with water, the rear surface portion gets swollen after the front surface portion starts to be swollen. As described above, the excluder body is swollen so as to be curved due to the restraint of the weld section. In this case, the front surface portion initially swollen tends to protrude outward, while the rear surface portion secondly swollen tends to be concaved, so as to form the curved shape.

In a case where the excluder body has a thickness (the width of the pressure applied surface) as small as 1 mm, there is generated substantially no temporal difference between the front surface portion to which water is supplied and the rear surface portion, with a result that any one of the surface portions may possibly protrude or be concaved. However, if the thickness is not less than 5 mm as mentioned above, the respective surface portions are swollen with a temporal difference. As a result, the direction of curvature can be controlled depending on which one of the surfaces is supplied with water by a user. In the case where such control is available, the excluder exerts further enhanced operability so as to be handled easily during a surgery.

Further reference is made to the case of the "flat plate shape with the center portion being widened after being swollen". In this case, the following features are preferably realized in the state where the excluder body is compressed and shaped.

More specifically, the excluder body has a bar shape when being compressed and shaped, when a longitudinal direction of the excluder body is referred to as a Z direction, one of directions perpendicular to the Z direction is referred to as an X direction, and a direction perpendicular to the Z direction and the X direction is referred to as a Y direction, the excluder body in the bar shape has a section that is perpendicular to the Z direction and has a rectangular shape, and side surfaces in the X direction and the Y direction, and the expansion stress is applied at least in the X direction, and the expansion stress is preferably higher in a center portion of the excluder body in the bar shape as compared to both end portions thereof (hereinafter, this condition may be referred to as an aspect [1]).

The excluder in the bar shape with the section in the rectangular shape is deformed into the flat plate shape with the center portion being widened after being swollen (see Fig. 3(c) or the like to be referred to later). However, the end portions have small widths, so that the end portion can be pinched with forceps to easily shift or turn the excluder. In this manner, the excluder exerts excellent operability in the body cavity. Furthermore, because the end portions are small in size, the excluder can be fixed by pressing the end portion into a narrow space between organs.

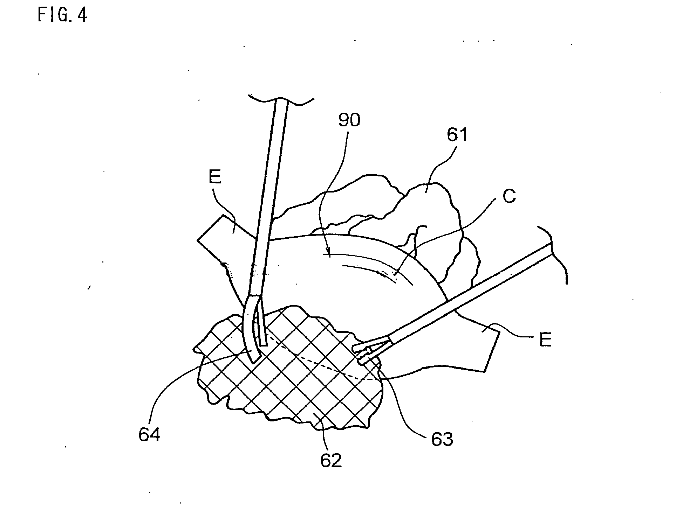

In some aspect of a surgery, the surgery may be performed in a state where an untargeted organ is excluded closer to an operator with use of the excluder, and a forceps, a knife, and the like are positioned so as to extend across the respective end portions of the excluder to reach the surgical field (see Fig. 4 to be referred to later). In such a case, the excluder having the "flat plate shape with the center portion being widened after being swollen" is capable of excluding the organ while the organ is securely covered with the center portion, and the forceps and the like can reach the surgical field by way of the narrow end portions, resulting in excellent operability during the surgery.

In the case of the aspect [1], if the compressed and shaped excluder in the bar shape has the transverse section (the XY plane) smaller than the section of the cylindrical inner cavity of the trocar, the excluder can be inserted into the body cavity through the cylinder of the trocar. When the excluder body is subsequently supplied with water so as to be swollen, the excluder body is grown to a size enough to exclude an organ.

Additionally, in the aspect [1], the weld section is preferably provided on each of the pressure applied surfaces upon compressing and shaping the excluder body in the bar shape so as to extend from one end to another end (hereinafter, this condition may be referred to as an aspect [1']).

In the aspect [1'], the excluder body in the bar shape is swollen to be deformed into a plate shape with the center portion being widened as compared to both the end portions. The excluder body is also pulled by the weld sections so as to be deformed into a boat bottom shape as a whole (see Figs. 3(a), 3(b), and the like to be referred to later). The excluder body in such a boat bottom shape is capable of excellently excluding an untargeted organ while the untargeted organ is covered with the excluder body. Furthermore, the excluder body has a three-dimensional structure in the boat bottom shape. Accordingly, the shape of the entire excluder can be kept more reliably with excellent and sustainable elasticity, thereby exerting satisfactory safety. Such a stable excluder realizes easier performance during a surgery, with a result of reduction in duration of the surgery.

### EFFECTS OF THE INVENTION

The surgical excluder according to the present invention can be accommodated in a small space for storage, and can be formed so as to be inserted through a trocar in an endoscopic surgery. At the same time, when being used in a surgery, the excluder can be swollen to be increased in size and can be deformed into a shape different from the shape before being swollen (in the compressed and shaped state). Therefore, the surgical excluder can be swollen so as to be deformed into a desired shape irrespective of the shape before being swollen.

### BRIEF DESCRIPTION OF THE DRAWINGS

Fig. 1 is a perspective view of a surgical excluder according to a first embodiment of the present invention.
Fig. 2 is a perspective view showing a state where the excluder according to the first embodiment is accommodated in a frame.
Fig. 3(a) is a perspective view showing a state where the surgical excluder according to the first embodiment is swollen, Fig. 3(b) is a sectional view seen along arrows D-D indicated in Figs. 3(a), and Fig. 3(c) is a plan view of only an excluder body being swollen.
Fig. 4 is an explanatory view showing a state where a surgery is being performed while an untargeted organ is excluded with use of the excluder according to the first embodiment.
Figs. 5(a) to 5(e) are perspective views showing a method (1) of producing the excluder according to the first embodiment, the views illustrating the respective steps of producing an endoscopic excluder in a bar shape from a block of cellulose sponge.
Figs. 6(a) to 6(f) are perspective views showing a method (2) of producing the excluder according to the first embodiment, the views illustrating the respective steps of producing an endoscopic excluder in a bar shape from a block of cellulose sponge.
Fig. 7(a) is a perspective view showing one step in a method (1) of producing a surgical excluder according to a second embodiment, Fig. 7(b) is a perspective view showing the subsequent step, and Fig. 7(c) is a perspective view showing the further subsequent step, illustrative of the surgical excluder according to the second embodiment.
Figs. 8(a) to 8(c) are perspective views showing a method (2) of producing the surgical excluder according to the second embodiment, the views illustrating the respective steps thereof.
Fig. 9(a) is a perspective view of a surgical excluder according to a third embodiment of the present invention, and Fig. 9(b) is a plan view showing a state where the excluder is swollen.
Figs. 10(a) to 10(e) are perspective views showing a method of producing the surgical excluder according to the third embodiment, the views illustrating the respective steps thereof.
Fig. 11(a) is a perspective view of a surgical excluder according to a fourth embodiment of the present invention, Fig. 11(b) is a perspective view showing a state where the excluder is swollen, and Fig. 11(c) is a plan view showing a state where only an excluder body of the excluder is swollen.
Fig. 12(a) is a perspective view of a surgical excluder according to a fifth embodiment of the present invention, and Fig. 12(b) is a plan view showing a state where the excluder is swollen.
Fig. 13(a) is a perspective view of a surgical excluder according to a sixth embodiment of the present invention, Fig. 13(b) is a perspective view showing a state where an excluder 20 is swollen, and Fig. 13(c) is a perspective view showing a state before an excluder body of the excluder is compressed and shaped.
Fig. 14(a) is a perspective view of a surgical excluder according to a seventh embodiment, and Fig. 14(b) is a perspective view showing a state where the excluder is swollen.

### MODES FOR CARRYING OUT THE INVENTION

### <First Embodiment>

Fig. 1 is a perspective view of a surgical excluder 90 according to a first embodiment of the present invention.

This excluder 90 is for use in an endoscopic surgery, and includes an excluder body 91 and weld sections 92 that are welded to the excluder body 91.

The excluder body 91 is made of cellulose sponge and has a bar shape with a square shape in section (8 mm x 8 mm x 200 mm), while an extrusion direction in a process of producing the cellulose sponge is aligned with the longitudinal direction (a Z direction) of the excluder body 91 (it is noted that the longitudinal length thereof is shorter than the actual size for convenience in the figure). Furthermore, one of directions perpendicular to the Z direction is referred to as an X direction, while a direction perpendicular to the Z direction and the X direction is referred to as a Y direction.

The excluder body 91 has side surfaces 91a, 91b, 91c, and 91d, and is compressed (arrows A) at an average compressibility of 10% from the side surfaces 91b and 91d along an YZ plane, and is dried subsequently. Accordingly, the excluder body has residual expansion stress in the X direction. The side surfaces 91b and 91d correspond to the "pressure applied surfaces" mentioned earlier.

This expansion stress is higher (stronger) in a center portion C of the excluder body 91 as compared to both end portions E thereof.

The weld sections 92 are made of a thermoplastic resin containing a roentgenopaque substance (such as barium sulfate). More specifically, examples of the thermoplastic resin include a polypropylene series resin containing barium sulfate kneaded thereinto, and a vinyl chloride resin or a silicon series resin containing barium sulfate kneaded thereinto.

The weld sections 92 each having a linear shape are provided in the longitudinal direction (the Z direction) on the side surfaces 91b and 91d (the pressure applied surfaces) of the excluder body 91, respectively. The weld sections are located in the centers in the width directions (the Y direction) of the side surfaces 91b and 91d.

Because the excluder body 91 is sized as detailed above, the excluder 90 can be inserted through a trocar having a cylindrical inner cavity in section (the inner cavity having a perfect circle shape) with a diameter of 12 mm, so as to be placed in a body cavity.

The excluder body 91 is swollen and increased in size when water is supplied thereto. Due to this property, the excluder before use may be slightly expanded by absorbing ambient moisture at the stage of sterilization treatment or storage. If the excluder body is expanded too much, the excluder body may be grown to be larger than the cylindrical inner cavity of the trocar and thus may not be inserted through the trocar.

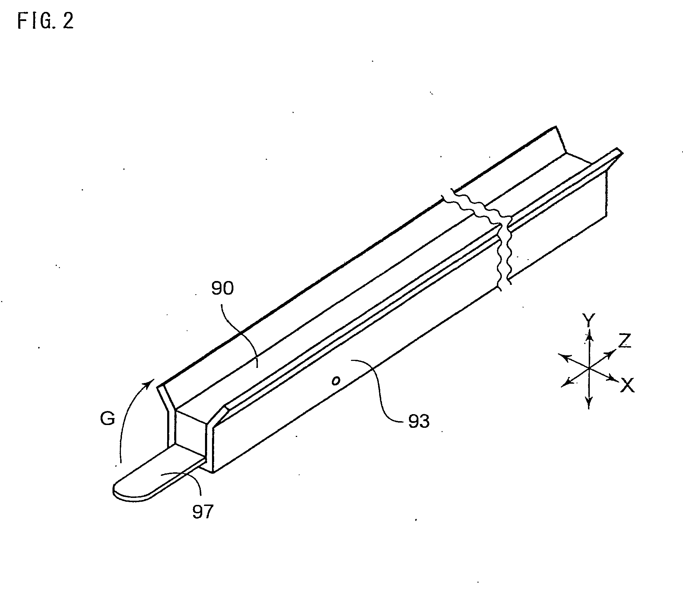

It is thus recommended to accommodate the excluder 90 in a frame 93, as shown in Fig. 2. This frame 93 suppresses expansion of the excluder, which can be kept in size so as to be inserted through the cylindrical inner cavity of the trocar. Fig. 2 also shows a tongue portion 97 that protrudes from one end in the longitudinal direction of the frame 93. When the tongue portion 97 is pulled upward (an arrow G), the excluder 90 is lifted upward and thus can be easily taken out of the frame 93.

Upon using the excluder 90 for an endoscopic surgery, the excluder 90 is taken out of the frame 93 and is inserted into the body cavity through the cylinder of the trocar. Subsequently, physiological saline or the like is poured on the excluder 90, which is thus swollen to exclude an untargeted organ.

Fig. 3(a) is a perspective view showing the state where the surgical excluder 90 according to the first embodiment is swollen. Fig. 3(b) is a sectional view seen along arrows D-D indicated in Fig. 3(a). Fig. 3(c) is a plan view showing a state where only the excluder body 91 (not provided with the weld sections 92) is swollen. The shape shown in Fig. 3(c) is identical with the shape of the excluder body 91 that is not yet compressed and shaped in the production process. Although there may be a case where the shape is not perfectly identical therewith because of residual stress in any of the processes of compression, storage, swelling, and the like, the shape can be restored so as to be substantially the same.

When supplied with water (such as physiological saline), the excluder 90 is swollen remarkably in the X direction in the center portion C that receives high expansion stress, and is swollen slightly in both the end portions E that receive low expansion stress (Figs. 1, 3(a), and 3(c)). Accordingly, in a case where the excluder 90 is not provided with the weld sections 92 (where only the excluder body 91 is prepared), as shown in Fig. 3(c), the excluder 90 is deformed into a flat plate shape with the center portion being large in width (W₂) and both the end portions being small in width (Wi). Outlined arrows F in Fig. 3(c) each indicate the direction of compression in the production process.

The excluder 90 according to the first embodiment is provided with the weld sections 92, which are not extended (expanded) by supply of water and thus serves to restrain swelling of the excluder body 91. Accordingly, the side surfaces 91b and 91d are pulled, while the center portion is swollen sufficiently, with a result that the excluder is curved into a boat shape as shown in Figs. 3(a) and 3(b).

In the example shown in Figs. 3(a) and 3(b), water is supplied onto the side surface 91a. In this case, the side surface 91c gets swollen after the side surface 91a has started to be swollen, so that the excluder is curved into a boat bottom shape while the side surface 91a protrudes outward and the side surface 91c is concaved.

Fig. 4 is an explanatory view showing a state where an operation is being performed while an intestine 61 (an untargeted organ) is excluded with use of the excluder 90 according to the first embodiment so as to enlarge a surgical field 62.

The excluder 90, which is curved into the boat bottom shape, can preferably exclude the intestine 61 so as to wrap the same.

In addition, the cellulose sponge (the water-absorbing dilatant material) as the material for the excluder body 91 of the excluder 90 exerts appropriate repulsive force and has a weight of a certain degree when being swollen, thereby being capable of preferably excluding the untargeted organ. Moreover, the swollen cellulose sponge is soft to be folded freely, so that such an excluder can be easily handled and gently exclude the untargeted organ.

Because the end portions E of the excluder 90 is small in width, the end portion E can be easily pinched and operated. Furthermore, the small end portion E can be interposed between organs. Moreover, as shown in Fig. 4, a first forceps 63 and a second forceps 64 can be inserted to cross over the low end portions E of the excluder 90 so as to control the surgical field 62. Therefore, the excluder 90 hardly disturbs the surgical operation.

In addition, because the weld sections 92 contain barium sulfate, it is also possible to check whether or not the excluder 90 remains by looking for the weld sections 92 through radiographic visualization (X-ray photography).

### <Water absorption test on excluder body>

The following test was conducted with regard to the water absorption rate of cellulose sponge, which is the material for the excluder body 91 according to the first embodiment.

The test was conducted in accordance with the falling-drop method 5.1.1 of the test on the water absorption rate in JIS L1907 5.1. More specifically, five test pieces each having a size of 30 mm x 30 mm were extracted from a specimen (test pieces Nos. 1 to 5). The position of a burette was adjusted such that the tip end of the burette is located higher by 10 mm than the surface of each of the test pieces. A drop of sugared water of a concentration of 65% was dripped from the burette, and measured with use of a stopwatch was a time period (second) from the drop of water reaching the surface of the test piece to the drop making no particular reflection.

Table 1 shows the test results on the water absorption rates. Gauze was selected as a specimen for comparison, and a similar test was conducted.

**[Table 1]**

| | Test piece No. 1 | Test Piece No.2 | Test Piece No.3 | Test Piece No.4 | Test Piece No.5 | Average |
|---|---|---|---|---|---|---|
| Cellulose sponge (Compressed to 1/5) | 4.56 | 4.73 | 4.62 | 6.00 | 6.34 | 5.25 |
| Cellulose sponge (Not compressed) | 9.69 | 6.85 | 7.34 | 6.36 | 9.00 | 7.85 |
| Gauze | 0.85 | 0.56 | 0.52 | 0.82 | 0.78 | 0.71 |
| (Unit: second) | | | | | | |

As proved by the results shown in Table 1, cellulose sponge absorbs water moment by moment such that water is dispersed gradually. Thus, in cellulose sponge having a thickness of a certain degree, water is not swollen simultaneously in a front surface portion, onto which water is supplied, and a rear surface portion. Table 1 also indicates that compressed cellulose sponge exerts a higher water absorption rate.

Amounts of water absorption of cellulose sponge were further tested in accordance with Notification No. 285 of the Ministry of Welfare, Standards for sanitary napkins, III Gauge and test method, (5) Amount of water absorption. More specifically, products of surgical excluders were selected as specimens (specimens Nos. 6 to 10, respectively). Each of the specimens was placed on a wire gauze having a known mass and 1, 680 µm (10 meshes). Water was gently poured onto the entire surface of the specimen from a beaker. Water was continuously poured until the entire specimen fully absorbed water and water further overflowed. The amount of water absorption was measured after each of the specimens was left for one minute. Table 2 shows the test results thereof.

**[Table 2]**

| | Specimen No.6 | Specimen No.7 | Specimen No.8 | Specimen No.9 | Specimen No. 10 | Average |
|---|---|---|---|---|---|---|
| Before water absorption (g) | 6.4 | 7.1 | 6.0 | 6.0 | 6.2 | 6.3 |
| After water absorption (g) | 163.8 | 176.3 | 158.9 | 152.7 | 157.1 | 161.7 |
| Amount of water absorption (g) | 157.4 | 169.2 | 152.9 | 146.6 | 150.9 | 155.4 |
| Scale factor of water absorption (times) | 24.7 | 23.9 | 25.5 | 24.3 | 24.4 | 24.6 |

As proved by the results indicated in Table 2, cellulose sponge exerts excellent water absorbability.

### <Verification test on direction of curvature of excluder>

Verification tests were conducted with use of the excluder 90 according to the first embodiment as to the direction of curvature when the excluder is swollen.

The tests were conducted in a state where the excluder 90 was placed on a board such that the side surface 91a or the side surface 91c (one of the surfaces not provided with the weld section 92) faces upward. Water was supplied from upward to swell the excluder, and the direction of curvature of the excluder was checked visually. The tests were conducted with use of ten specimens (specimens Nos. 11 to 20). Table 3 shows the results thereof.

**[Table 3]**

| | State |
|---|---|
| Specimen No. 11 | Convex upward |
| Specimen No. 12 | Convex upward |
| Specimen No. 13 | Convex upward |
| Specimen No. 14 | Convex upward |
| Specimen No. 15 | Convex upward |
| Specimen No. 16 | Convex upward |
| Specimen No. 17 | Convex upward |
| Specimen No. 18 | Convex upward |
| Specimen No. 19 | Convex upward |
| Specimen No. 20 | Convex upward |

As proved from Table 3, all the ten specimens were swollen and curved such that the surfaces supplied with water protruded outward and the surfaces opposite thereto were concaved. It is thus proved that the direction of curvature can be controlled by means of the operation of supplying water.

### <Production method (1) of excluder 90 according to first embodiment>

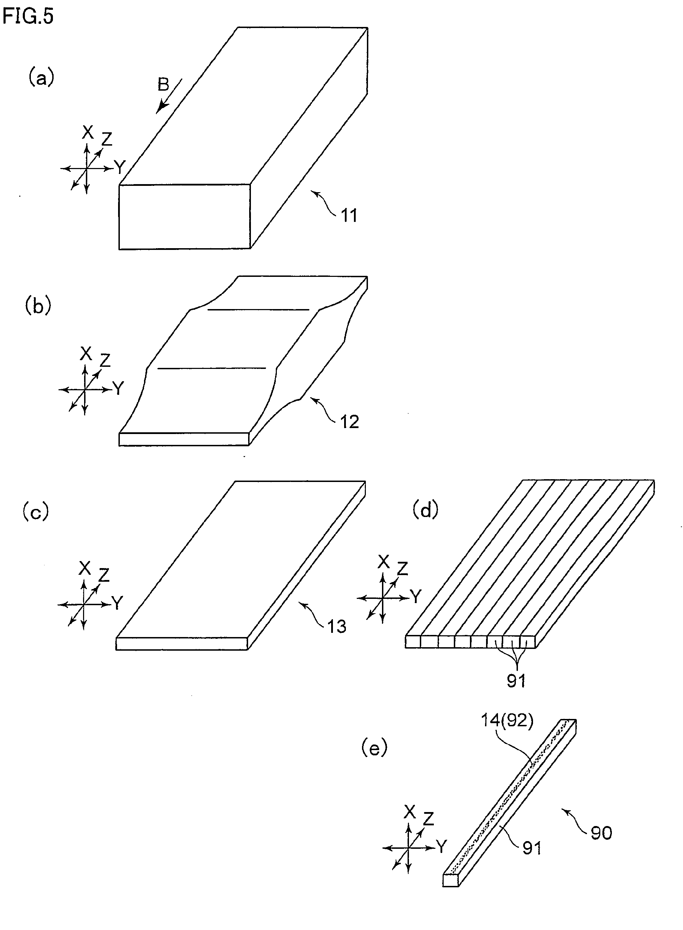

Described next is a method of producing the excluder 90 according to the first embodiment. Figs. 5(a) to 5(e) are perspective views showing the steps until obtaining an endoscopic excluder from a block of cellulose sponge.

The cellulose sponge as the material for the excluder body 91 can be produced in accordance with a cellulose regeneration method, a cellulose solvent solution method, or the like. For example, an example of such cellulose sponge is disclosed in Japanese Patent No. 3520511.

In specific exemplification of the method of producing cellulose sponge, initially, viscose is obtained from a cellulosic material such as wood chips. Natural fibers are added to the viscose, and further mixed thereinto are crystallized mirabilite, a polyol compound, and a surfactant if appropriate. Sponge liquid concentrate thus obtained is extruded and filled into a mold tool to be then heated and solidified, so as to obtain a block of cellulose sponge (Fig. 5(a); an arrow B in the figure indicates an extrusion direction).

A cellulose sponge block 11 thus obtained is cut such that both end portions are narrowed in the X direction, as shown in Fig. 5(b) (a cut block 12). The cut block 12 is dried while being compressed in the X direction (it is noted that the direction of compression is consistent with the directions indicated by the arrows A), so as to be formed into a flat plate shape (Fig. 5(c)). In some cases, the cut block may be compressed also in the Y direction or the Z direction. In such a case where the cut block is compressed in the Y direction or the Z direction, the cut block is expanded also in the Y direction or the Z direction by supply of water.

A flat plate object 13 is then sliced in the Z direction to produce a plurality of excluder bodies 91 each in a bar shape (having a section in a rectangular shape) (Fig. 5(d)).

Meanwhile, multifilaments are produced by kneading barium sulfate into a polypropylene series resin, and are collected into a thread shape (which is referred to as a contrast thread 14). The contrast thread 14 is disposed on each of the compressed surfaces (the pressure applied surfaces) of the excluder body 91 at the center of the width (the center in the Y direction) in the longitudinal direction (the Z direction), and is heated for pressure joining, so that the contrast thread 14 is welded to form the weld section 92 (Figs. 5(e) and 1). According to the example shown in Figs. 5(a) to 5(e), there are obtained eight excluders 90, while Fig. 5(e) shows only one of the excluders 90.

### <Production method (2) of excluder 90 according to first embodiment>

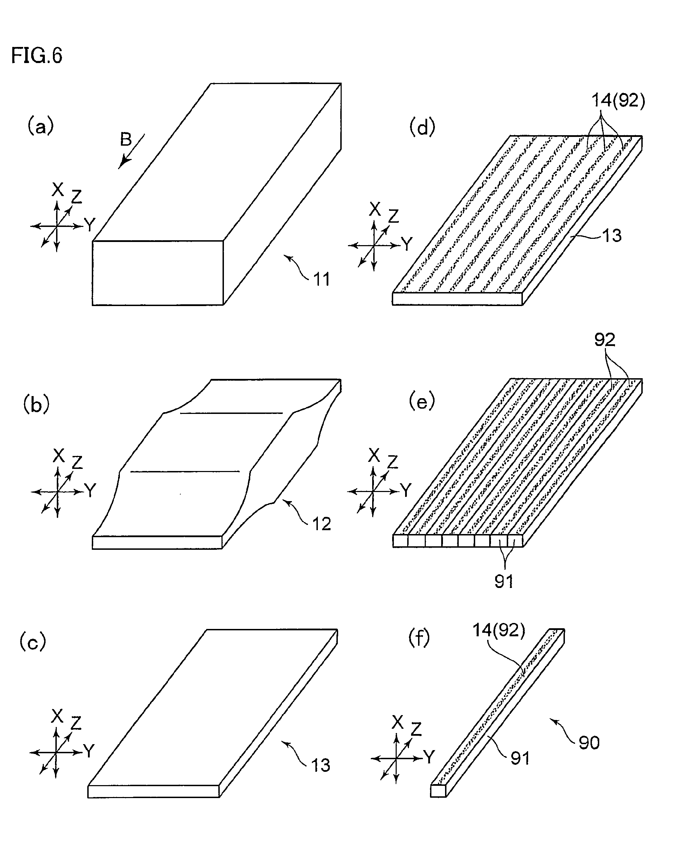

Figs. 6(a) to 6(f) are perspective views showing a method (2) of producing the excluder 90 according to the first embodiment, the views illustrating the respective steps thereof.

The steps of producing a cut block 12 using cellulose sponge 11 as the material therefor to drying the cut block while compressing in the X direction so as to form into a flat plate shape (a flat plate object 13) are the same as those steps illustrated in Figs. 5(a) to 5(c) (Figs. 6(a) to 6(c)).

Subsequently, as shown in Fig. 6(d), a predetermined number (eight in the example shown in the figure) of contrast threads 14 are disposed in the Z direction so as to be spaced apart from one another, on both the compressed surfaces (the pressure applied surfaces) of the flat plate object 13. The flat plate object is heated for pressure joining, so that the contrast threads 14 are welded to form the weld sections 92.

The flat plate object 13 is then cut at positions between the adjacent weld sections 92 (Fig. 6(e)), so as to obtain a plurality of excluders 90 each in a bar shape (having a section in a rectangular shape) (Fig. 6(f) showing only one of the excluders 90, and Fig. 1).

### <Second Embodiment>

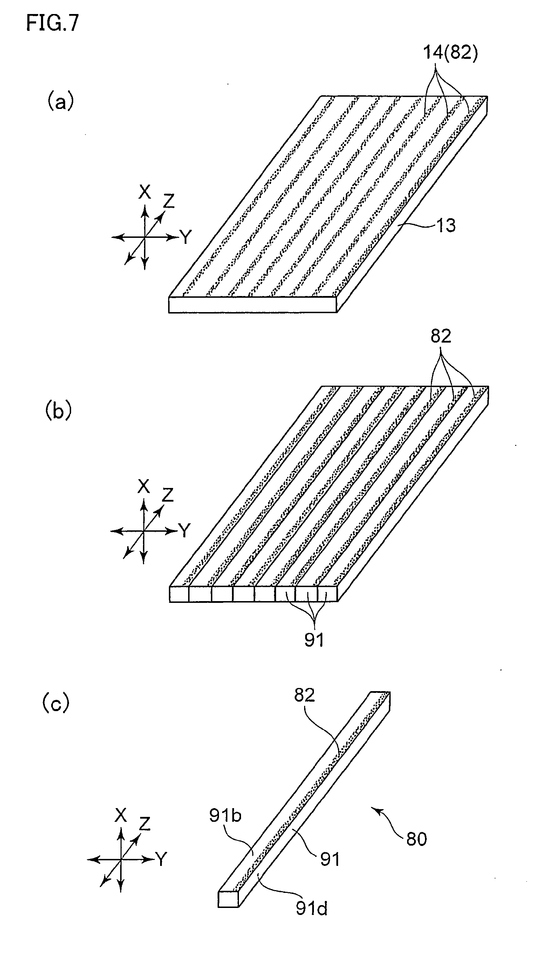

Fig. 7(c) is a perspective view of a surgical excluder 80 according to a second embodiment of the present invention.

The excluder 90 according to the first embodiment is provided with the weld sections 92 that are located at the centers of the width (the Y direction) on the side surfaces 91b and 91d (the pressure applied surfaces) of the excluder body 91. To the contrary, the excluder 80 according to the second embodiment is provided with weld sections 82 that are disposed close to edges of corresponding side surfaces of the excluder body 91.

In the excluder 90 according to the first embodiment, the direction of concavity into the boat bottom shape is controlled depending on to which one of the side surfaces 91a and 91c water is supplied for use. However, if water is supplied to the side surface 91b or 91d on which the weld section is provided or to the tip end of the excluder 90 in the bar shape, the direction of concavity into the boat bottom shape is not predetermined with respect to the Y axis but the excluder is concaved in one of the directions due to a slight difference in the swollen state. As described above, the excluder 80 according to the second embodiment is provided with the weld sections 82 that are not located at the centers of the side surfaces 91b and 91d. In this configuration, the portions provided with the weld sections 82 are pulled more intensively, so that the excluder is concaved in the direction opposite thereto.

### <Production method (1) of excluder 80 according to second embodiment>

Figs. 7(a) and 7(b) are perspective views showing the respective steps in a method (1) of producing the excluder 80 according to the second embodiment. Fig. 7(c) is a perspective view of the completed excluder 80.

The steps of producing a cut block 12 using cellulose sponge 11 as the material therefor to drying the cut block while compressing in the X direction so as to form into a flat plate shape are the same as those steps illustrated in Figs. 5(a) to 5(c).

Subsequently, as shown in Fig. 7(a), a predetermined number of contrast threads 14 are disposed in the Z direction so as to be spaced apart from one another, on both the compressed surfaces (the pressure applied surfaces) of the flat plate object 13. In this step, in consideration of the positions to be cut to obtain the bar shapes in a later step, the contrast threads 14 are disposed adjacent to the cut positions, respectively. The flat plate object is heated for pressure joining, so that the contrast threads 14 are welded to form the weld sections 82.

The flat plate object 13 is then cut at positions close to the weld sections 82 (Fig. 7(b)), so as to obtain a plurality of excluders 80 each in a bar shape (having a section in a rectangular shape) (Fig. 7(c) showing only one of the excluders 80).

### <Production method (2) of excluder 80 according to second embodiment>

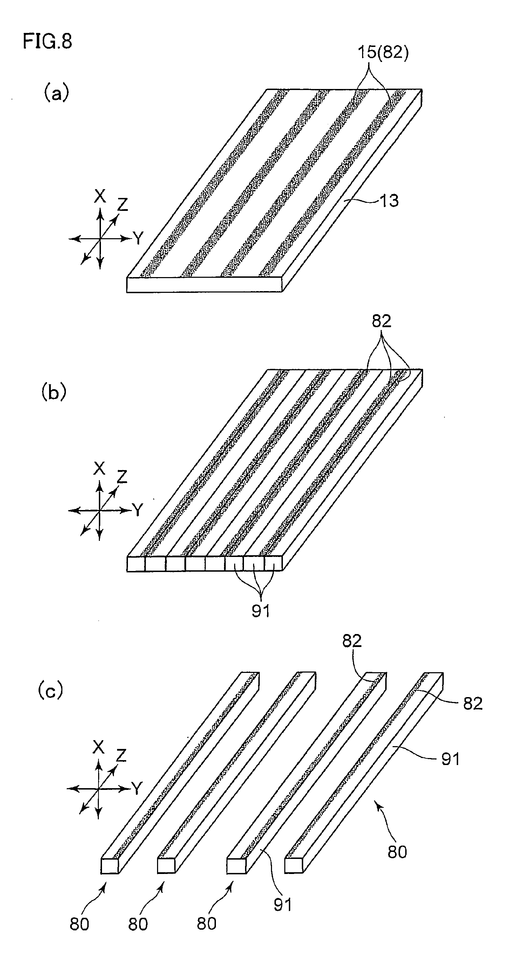

Figs. 8(a) to 8(c) are perspective views showing a method (2) of producing the excluder 80 according to the second embodiment, the views illustrating the respective steps thereof.

The steps of producing a cut block 12 using cellulose sponge 11 as the material therefor to drying the cut block while compressing in the X direction so as to form into a flat plate shape are the same as those steps illustrated in Figs. 5(a) to 5(c).

Subsequently, two contrast threads 14 are parallelly combined into a set (paired contrast threads 15). As shown in Fig. 8(a), the paired contrast threads 15 are disposed in the Z direction on both the compressed surfaces (the pressure applied surfaces) of the flat plate object 13. In this step, the paired contrast threads 15 are disposed at every other position to be cut to obtain the bar shapes in a later step, so as to be located across the position to be cut. The flat plate object is heated for pressure joining, so that the paired contrast threads 15 are welded to form the weld sections 82.

Subsequently, as shown in Fig. 8(b), the flat plate object is cut at the center of each of the weld sections 82 as well as at positions between the adjacent weld sections 82. The weld sections 82 are each cut at the center thereof into two portions, each of which is disposed on corresponding one of the excluders 80 (Fig. 8(c)). According to the example shown in Figs. 8(a) to 8(c), there are obtained eight excluders 80, while Fig. 8(c) shows only four of the excluders 80. Obtained in this manner are the plurality of excluders 80 each in a bar shape (having a section in a rectangular shape).

### <Third Embodiment>

Fig. 9(a) is a perspective view of a surgical excluder 70 according to a third embodiment of the present invention, and Fig. 9(b) is a plan view showing a state where the excluder 70 is swollen.

The excluder 70 according to the third embodiment is configured only by an excluder body 71 that is made of cellulose sponge, and is not provided with any weld section.

The excluder 70 has a bar shape with a section in a rectangular shape in the state before being swollen (the compressed and shaped state) (Fig. 9(a)). The excluder body 71 of the excluder 70 is compressed (arrows F) at an average compressibility of 10% from side surfaces (pressure applied surfaces) facing the X direction, and is then dried. Accordingly, the excluder body has residual expansion stress in the X direction.

When supplied with water (such as physiological saline), the excluder 70 is swollen in the X direction. In the state before being swollen (the compressed and shaped state), the expansion stress has a low value at a position provided with a cutout section 73 and has a high value at positions 74 on both sides of the cutout section 73.

With use of the excluder 70, upon a sigmoidal surgery or the like, a descending colon can be fitted in the cutout section 73 so that the excluder 70 can exclude the upper colons and the small intestine. As described above, the swollen excluder can be deformed into a different shape suitable for a surgery. On the other hand, the excluder not yet swollen has the bar shape and thus can be accommodated in a small space for storage. In addition, the excluder can be used as an endoscopic excluder that is shaped so as to be inserted through a trocar.

### <Production method of excluder 70 according to third embodiment>

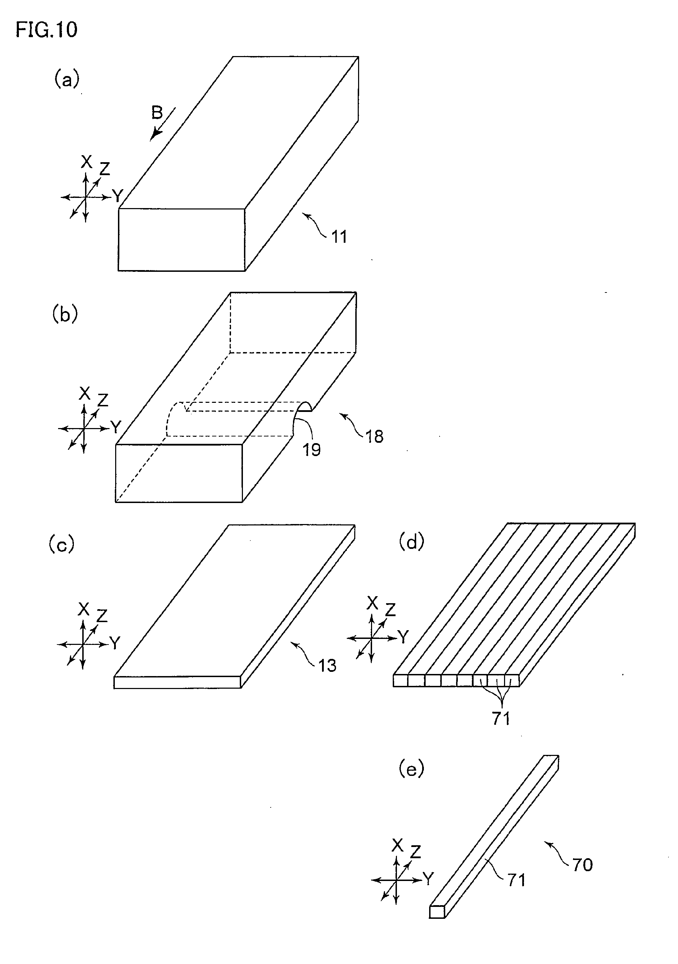

Figs. 10(a) to 10(e) are perspective views showing a method of producing the excluder 70 according to the third embodiment, the views illustrating the respective steps thereof.

The step of producing a block of cellulose sponge 11 as the material is the same as the step shown in Fig. 5(a) (Fig. 10(a)).

Subsequently, as shown in Fig. 10(b), the cellulose sponge 11 is cut out so as to form a concavity 19 (a cut block 18). Then, the cut block is dried while being compressed in the X direction so as to be formed into a flat plate shape (a flat plate object 13) (Fig. 10(c)).

Thereafter, similarly to the case in the production method (1) according to the first embodiment, the flat plate object 13 is sliced in the Z direction (Fig. 10(d)) to obtain a plurality of excluder bodies 71 (the excluders 70) each in a bar shape (Fig. 10(e)).

### <Other Embodiments>

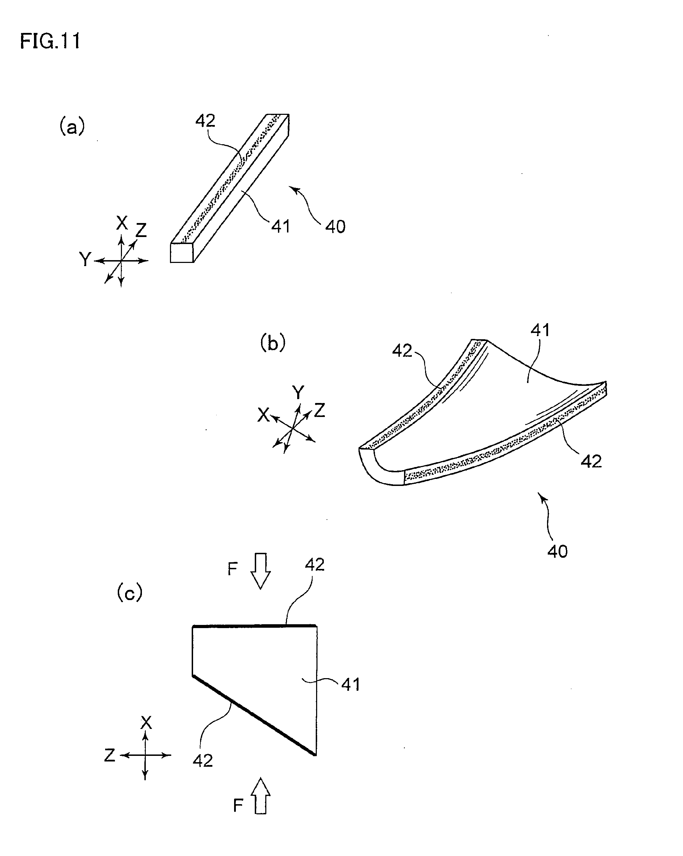

Figs. 11(a) to 11(c) are views of a surgical excluder 40 according to a fourth embodiment of the present invention. More specifically, Fig. 11(a) is a perspective view of the excluder 40, and Fig. 11(b) is a perspective view showing a state where the excluder 40 is swollen. Fig. 11(c) is a plan view showing a state where only an excluder body 41 of the excluder 40 is swollen, in which positions of weld sections 42 are also indicated for the purpose of easier comprehension. Outlined arrows F in Fig. 11(c) each indicate the direction of compression when the excluder is compressed and shaped.

In the excluder 40, the swollen excluder body 41 has a halved trapezoid shape obtained by cutting off an apex of a right angled triangle (Fig. 11(c)), and the weld sections 42 are provided on lateral edges thereof. Accordingly, the excluder being swollen is restrained by the weld sections 42 and is thus curved (Fig. 11(b)).

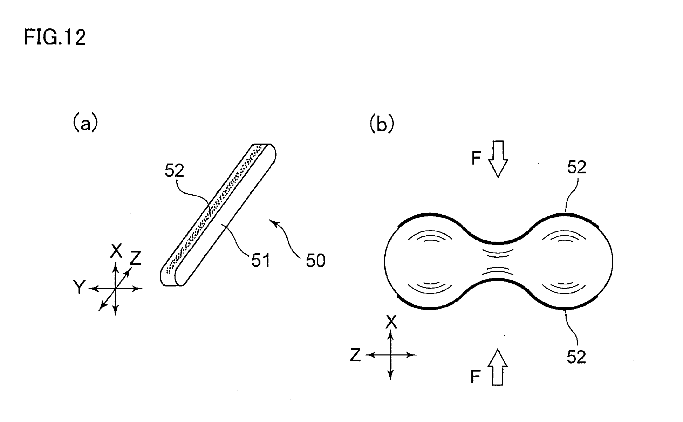

Figs. 12(a) and 12(b) are views of a surgical excluder 50 according to a fifth embodiment of the present invention. More specifically, Fig. 12(a) is a perspective view of the excluder 50, and Fig. 12(b) is a plan view showing a state where the excluder 50 is swollen. Outlined arrows F in Fig. 12(b) each indicate the direction of compression when the excluder is compressed and shaped.

The excluder 50 according to the fifth embodiment includes an excluder body 51 that has a gourd shape and has side surfaces provided weld sections 52. When being swollen, the excluder 50 is curved into a gourd shape that has two recesses.

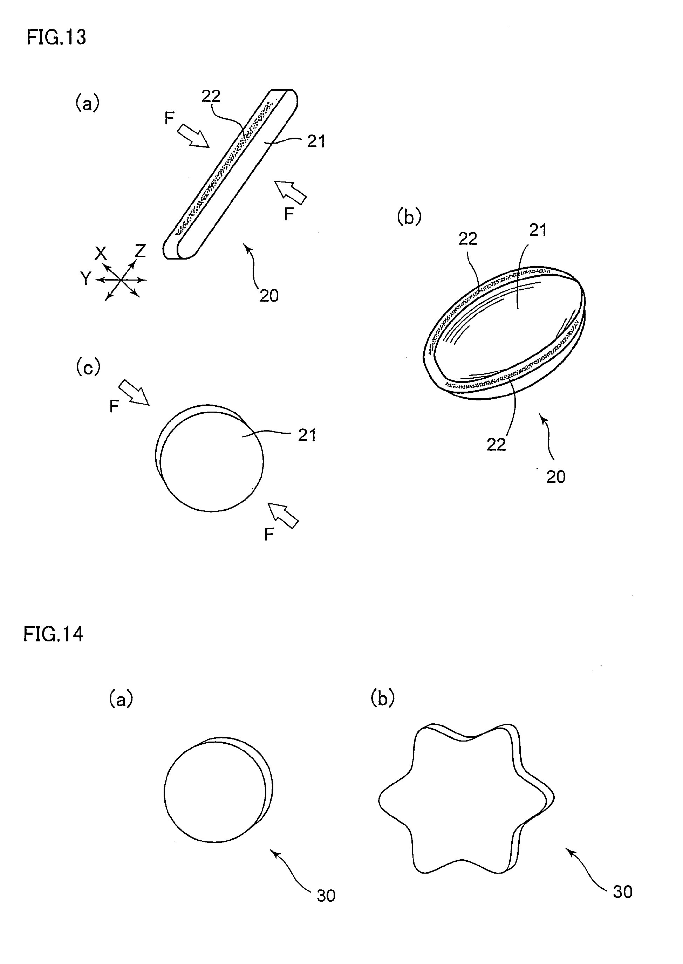

Figs. 13(a) to 13(c) are views of a surgical excluder 20 according to a sixth embodiment of the present invention. More specifically, Fig. 13(a) is a perspective view of the excluder 20, and Fig. 13(b) is a perspective view showing a state where the excluder 20 is swollen. Fig. 13(c) is a perspective view showing a state before an excluder body 21 of the excluder 20 is compressed. Outlined arrows F in Figs. 13(a) and 13(c) each indicate the direction of compression when the excluder is compressed and shaped.

The excluder 20 according to the sixth embodiment is obtained by compressing the excluder body 21 in a disc shape from peripheral side surfaces (in the F or X direction in Fig. 13(c)), and weld sections 22 are provided on both of the pressure applied surfaces (Fig. 13(a)). The excluder 20 being swollen is restrained by the weld sections 22 and is thus curved into a deeply concaved boat shape (Fig. 13(b)).

Fig. 14(a) is a perspective view of a surgical excluder 30 according to a seventh embodiment of the present invention, and Fig. 14(b) is a perspective view showing a state where the excluder 30 is swollen.

The excluder 30 according to the seventh embodiment is not provided with any weld section, and includes only an excluder body made of cellulose sponge. The disc shape before being swollen (Fig. 14(a)) is deformed into a star shape provided with six projections when being swollen (Fig. 14(b)).

The present invention has been specifically described by way of examples. However, the present invention should not be limited to these examples, and can be also embodied with appropriate modifications as long as being applicable to the purposes of the invention. All of such modifications are to be included in the technical scope of the present invention.

The above embodiments exemplify cellulose sponge as a material for the excluder body. However, the present invention is not limited to such a case, but the material therefor may be a water-absorbing dilatant material such as polyvinyl alcohol or polyurethane.

The first embodiment and the like each exemplify the configuration in which the weld sections are provided on both the surfaces in the direction of compression. Alternatively, there may be provided only one weld section on one of the surfaces in the direction of compression. Furthermore, the weld section may not be limited to the linear shape as exemplified in the above embodiments. Alternatively, the weld section may have a circular arc line shape, a zigzag line shape, a waved line shape, or the like. Still alternatively, the weld section is not limited to such a linear shape.

The above first to sixth embodiments each exemplify the excluder before being swollen having the bar shape. However, the present invention is not limited to such a case. Alternatively, the excluder may be in the disc shape as exemplified in the seventh embodiment. Still alternatively, the excluder may have a spherical shape before being swollen, and may be swollen such that projections protrude to form a large number of bulges when water is supplied thereto. In these manners, the excluder may have various shapes both before and after being swollen.

### INDUSTRIAL APPLICABILITY

The present invention is applicable in various surgeries such as an endoscopic surgery and laparotomy (inclusive of thoracotomy).

### DESCRIPTION OF REFERENCE SIGNS

20, 30, 40, 50, 70, 80, 90 Excluder
21, 41, 51, 71, 91 Excluder body
22, 42, 52, 72, 82, 92 Weld section
61 Intestine
62 Surgical field
63 First forceps
64 Second forceps
91a, 91b, 91c, 91d Side surface

## Claims

1. A surgical excluder to be inserted into a body during a surgery, the excluder comprising:
an excluder body configured by compressing and shaping a water-absorbing dilatant material so that expansion stress in a direction opposite to a direction of compression remains, the residual expansion stress having values varied from portion to portion.

2. The surgical excluder according to claim 1, further comprising a weld section made of a thermoplastic resin on at least one of pressure applied surfaces to which pressure is applied upon compressing and shaping the excluder body, the weld section restraining the excluder body.

3. The surgical excluder according to claim 2, wherein the pressure applied surfaces of the excluder body each have a width not less than 5 mm.

4. The surgical excluder according to any one of claims 1 to 3,
wherein
the excluder body has a bar shape when being compressed and shaped,
when a longitudinal direction of the excluder body is referred to as a Z direction, one of directions perpendicular to the Z direction is referred to as an X direction, and a direction perpendicular to the Z direction and the X direction is referred to as a Y direction,
the excluder body in the bar shape has a section that is perpendicular to the Z direction and has a rectangular shape, and side surfaces in the X direction and the Y direction, and
the expansion stress is applied at least in the X direction, and the expansion stress is higher in a center portion of the excluder body in the bar shape as compared to both end portions thereof.

5. The surgical excluder according to claim 4, wherein the weld section is provided on each of the pressure applied surfaces upon compressing and shaping the excluder body in the bar shape so as to be provided over one end to another end.
